# EUROPEAN PATENT SPECIFICATION

(11) **EP 3 576 076 B1**
(45) Date of publication and mention of the grant of the patent: **06.01.2021**
(21) Application number: 18742259.7
(22) Date of filing: 18.01.2018
(51) Int. Cl.: G09B 9/00, G09B 23/28, A61B 1/00, G09B 23/30

(54) **ENDOSCOPIC PROCEDURE SIMULATOR MODULE AND ENDOSCOPIC PROCEDURE SIMULATOR USING SAME**
SIMULATORMODUL FÜR ENDOSKOPISCHES VERFAHREN UND SIMULATOR FÜR ENDOSKOPISCHES VERFAHREN DAMIT
MODULE DE SIMULATEUR DE PROCÉDURE ENDOSCOPIQUE ET SIMULATEUR DE PROCÉDURE ENDOSCOPIQUE L'UTILISANT

(30) Priority: 20.01.2017 KR 20170010033
(43) Date of publication of application: 04.12.2019
(73) Proprietor: The Asan Foundation, Seoul 05505 (KR); University Of Ulsan Foundation For Industry Cooperation, Ulsan 44610 (KR)
(72) Inventor: AHN, Ji Yong, Seoul 06006 (KR); LEE, Gin Hyug, Seoul 05571 (KR)
(74) Representative: Becker & Kurig Partnerschaft Patentanwälte PartmbB
(86) International application number: PCT/KR2018/000851
(87) International publication number: WO 2018/135879

(56) References cited:
- WO-A1-2010/016353
- WO-A1-2016/044577
- JP-A- 2001 005 377
- JP-A- 2008 197 483
- JP-A- 2008 197 483
- JP-A- 2015 125 231
- JP-A- 2015 125 231
- KR-A- 20140 084 053
- KR-A- 20160 135 267
- US-A1- 2005 008 997
- CHEN P C ET AL: "Hemostatic effect of endoscopic local injection with hypertonic saline-epinephrine solution and pure ethanol for digestive tract bleeding", GASTROINTESTINAL ENDOSCOPY, ELSEVIER, NL, vol. 32, no. 5, 1 October 1986 (1986-10-01), pages 319-323, XP025856952, ISSN: 0016-5107, DOI: 10.1016/S0016-5107(86)71875-0 [retrieved on 1986-10-01]

## Description

### BACKGROUND

Embodiments of the inventive concept described herein relate to an endoscopic procedure simulator module and an endoscopic procedure simulator using the same, and more particularly, relate to an endoscopic procedure simulator module for presenting a lesion state and enabling optimal training for an endoscopic procedure, and an endoscopic procedure simulator using the same.

In general, endoscopic procedures are performed by inserting an endoscope having a camera installed therein and various types of procedure instruments through a small hole such as an oral cavity or an anus without a large incision in a human body and examining a diseased part by using images obtained through the endoscope. Most of the endoscopic procedures are performed through an oral cavity or an anus and therefore have advantages of no incision and scarring of the skin and fast recovery time, compared with laparotomy. With the development of endoscopes and instruments, the endoscopic procedures have evolved to a degree that the endoscopic procedures can treat many of diseases for which laparotomy was required in the past, and the endoscopic procedures have been increasingly applied to other medical fields.

For example, an endoscope may be inserted through an oral cavity and used to examine a throat and a duodenum and, when necessary, may be inserted into a small intestine and used to examine the small intestine. Alternatively, the endoscope may be inserted into a large intestine through an anus and used to examine, diagnose, or treat the interior of each organ by using images obtained through a camera mounted in the endoscope. In many cases, diseases generated in the interior of an organ may be diagnosed through the endoscope. In addition, treatments such as stopping bleeding, cutting an early cancer or a polyp, anastomosis of a fistula, and the like may be performed through the endoscope.

The endoscope has a long tubular shape. The endoscope includes a camera channel into which a camera is inserted, a working channel into which a pair of forceps for a biopsy, a syringe needle, and an electric knife for cutting a lesion are inserted and moved, and a suction channel for removing foreign matter generated from a diseased part.

However, an unskilled operator may cause unexpected problems (e.g., inaccurate diagnosis, a failure in hemostasis, a failure to remove an appropriate tumor, bleeding, perforation, and the like) due to poor manipulation in the process of performing a procedure while moving the endoscope into an organ for diagnosis or treatment through the endoscope.

Accordingly, in manipulating an endoscope while inserting and moving the endoscope into an organ, an endoscopic procedure simulator module for performing training in endoscope manipulation and endoscopic procedure in response to various lesion phenomena and an endoscopic procedure simulator using the same are required. JP 2015125231 A discloses a simulated mucosal tissue of an endoscope remedy training model including a simulated mucosal lower layer and a simulated muscle layer. WO 2016044577 A1 discloses methods for creating a pressurized cadaver model used for surgical procedure training, wherein the internal jugular veins, common carotid arteries, brachial arteries, superficial femoral arteries and femoral veins thereof of a cadaver are exposed

### SUMMARY

Embodiments of the inventive concept provide an endoscopic procedure simulator module for repeatedly performing training in endoscope manipulation and endoscopic procedure in response to various lesion phenomena, and an endoscopic procedure simulator using the same.

According to the claimed invention an endoscopic procedure simulator module includes a lesion indicating part having a discharge hole formed therein, through which a fluid is discharged to present a bleeding state, a module body to which the lesion indicating part is coupled, the module body having a fluid channel and a spacing space formed therein, in which the fluid to be discharged through the discharge hole flows through the fluid channel and the spacing space is separated from the fluid channel, and a diaphragm provided in the spacing space of the module body so as to be expandable, in which the diaphragm is expanded by a separate fluid injected into the spacing space and closes the discharge hole of the lesion indicating part, wherein the lesion indicating part has a syringe needle passage portion that is formed around the discharge hole thereof and through which a syringe needle that injects the separate fluid into the spacing space passes.

The fluid channel may be partitioned into a lower fluid channel and an upper fluid channel by a partition plate, the partition plate having an upper inlet formed therein, through which part of the fluid flowing through the lower fluid channel is introduced into the upper fluid channel.

The module body may have an inlet and an outlet that are formed therein and connected by a tube, the fluid being introduced into the lower fluid channel through the inlet and discharged from the lower fluid channel through the outlet.

According to an exemplary embodiment, an endoscopic procedure simulator module includes a lesion indicating part having a plurality of protrusions to present a polyp, each protrusion having a discharge hole formed therein, through which a fluid is discharged and a module body to which the lesion indicating part is coupled, the module body having a fluid channel formed therein, in which the fluid to be discharged through the discharge hole flows through the fluid channel.

The endoscopic procedure simulator module may further include a distribution member that is provided between the lesion indicating part and the module body and that distributes the fluid discharged from the fluid channel of the module body to the discharge holes of the plurality of protrusions.

The module body may have an inlet and an outlet that are formed therein and connected by a tube, the fluid being introduced into the fluid channel through the inlet and discharged from the fluid channel through the outlet.

According to an exemplary embodiment, an endoscopic procedure simulator module includes a lesion indicating part having a plurality of protrusions to present a polyp and having an electric wire electrically coupled to one side thereof, the lesion indicating part being formed of a conductive material and a module body to which the lesion indicating part is coupled such that the plurality of protrusions are exposed.

The module body may include a lower module body in a rectangular block shape on which the lesion indicating part and a terminal are seated and an upper module body protruding from the lower module body and having an arc shape to receive the lesion indicating part therein.

According to an exemplary embodiment, an endoscopic procedure simulator includes a model organ having the shape of an organ and including an insertion space formed therein and one or more coupling holes formed through a surface thereof, in which an endoscope is inserted and moved into the insertion space and the one or more coupling holes communicate with the insertion space, and the module detachably coupled to the coupling hole of the model organ such that the lesion indicating part is exposed to the insertion space.

The endoscopic procedure simulator may further include a fixing frame that surrounds and fixes the model organ.

### BRIEF DESCRIPTION OF THE FIGURES

The above and other objects and features will become apparent from the following description with reference to the following figures, wherein like reference numerals refer to like parts throughout the various figures unless otherwise specified, and wherein:
FIG. 1 is a perspective view of an endoscopic procedure simulator module according to a first embodiment of the inventive concept;
FIG. 2 is a sectional view illustrating a state in which a fluid is discharged through a discharge hole of the endoscopic procedure simulator module of FIG. 1;
FIG. 3 is a sectional view illustrating a state in which no fluid is discharged through the discharge hole of the endoscopic procedure simulator module of FIG. 1;
FIG. 4 is a perspective view of an endoscopic procedure simulator module according to a second embodiment of the inventive concept;
FIG. 5 is an exploded perspective view of FIG. 4;
FIG. 6 is a sectional view of FIG. 4;
FIG. 7 is a perspective view of an endoscopic procedure simulator module according to a third embodiment of the inventive concept;
FIG. 8 is a sectional view of FIG. 7;
FIG. 9 is a perspective view illustrating a procedure state of the endoscopic procedure simulator module according to the third embodiment of the inventive concept;
FIG. 10 is a sectional view of FIG. 9;
FIG. 11 is a perspective view of an endoscopic procedure simulator according to an embodiment of the inventive concept; and
FIG. 12 is a partial enlarged perspective view of the interior of a model organ in FIG. 11, where FIG. 12 illustrates a state in which the modules are mounted in the model organ.

### DETAILED DESCRIPTION

The above and other aspects, features, and advantages of the inventive concept will become apparent from the following description of embodiments given in conjunction with the accompanying drawings. However, the inventive concept is not limited to the embodiments disclosed herein and may be implemented in various different forms. Herein, the embodiments are provided to provide complete disclosure of the inventive concept and to provide thorough understanding of the inventive concept to those skilled in the art to which the inventive concept pertains.

Terms used herein are only for description of embodiments and are not intended to limit the inventive concept. As used herein, the singular forms are intended to include the plural forms as well, unless context clearly indicates otherwise. It will be further understood that the terms "comprise" and/or "comprising" specify the presence of stated features, components, and/or operations, but do not preclude the presence or addition of one or more other features, components, and/or operations. In addition, identical numerals will denote identical components throughout the specification, and the meaning of "and/or" includes each mentioned item and every combination of mentioned items.

Unless otherwise defined, all terms (including technical and scientific terms) used herein have the same meaning as commonly understood by those skilled in the art to which the inventive concept pertains. It will be further understood that terms, such as those defined in commonly used dictionaries, should not be interpreted in an idealized or overly formal sense unless expressly so defined herein.

Hereinafter, the inventive concept will be described in detail with reference to the accompanying drawings.

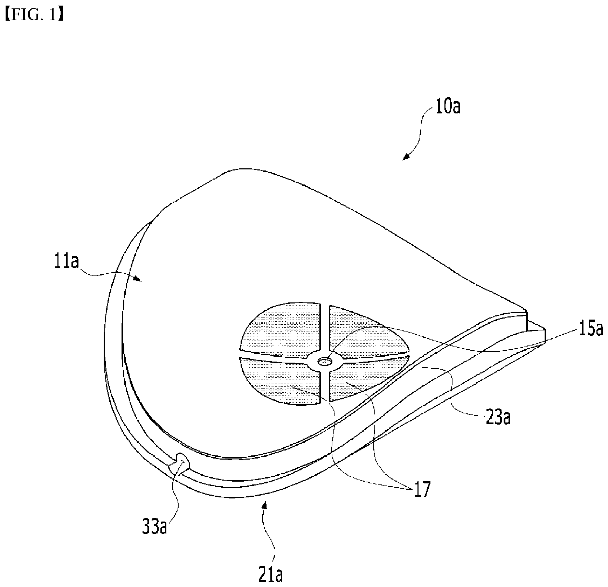

FIGS. 1 to 3 illustrate an endoscopic procedure simulator module according to a first embodiment of the inventive concept.

As illustrated in these drawings, the endoscopic procedure simulator module 10a according to the first embodiment of the inventive concept includes a lesion indicating part 11a, a module body 21a, and a diaphragm 45.

The lesion indicating part 11a has a disc shape that is concavely formed to be curved to one side. The lesion indicating part 11a has, in the central region thereof, a discharge hole 15a through which a fluid is discharged. The lesion indicating part 11a may present a bleeding state as the fluid is discharged through the discharge hole 15a. Although the lesion indicating part 11a in this embodiment is illustrated as being concavely formed to be curved to the one side, the lesion indicating part 11a, without being limited thereto, may protrude so as to be curved to the one side or may be formed to be flat.

The lesion indicating part 11a further includes a syringe needle passage portion 17 through which a syringe needle for injecting a separate fluid into a spacing space 41 that will be described below passes. The syringe needle passage portion 17 is provided around the discharge hole 15a of the lesion indicating part 11a. In this embodiment, four syringe needle passage portions 17 are formed around the discharge hole 15a at equal intervals. Without being limited thereto, however, one or more syringe needle passage portions 17 may be provided around the discharge hole 15a.

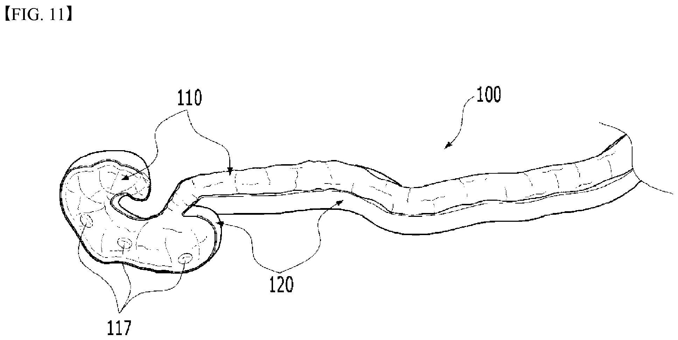

The module body 21a has a disc shape. The module body 21a has, on the periphery thereof, a coupling part 23a with a reduced diameter. The coupling part 23a is fit into a coupling hole 117 (refer to FIG. 11) of an endoscopic procedure simulator 100 (refer to FIG. 11), which will be described below. Accordingly, the module body 21a is coupled to the endoscopic procedure simulator 100.

The module body 21a has a fluid channel 25a formed therein, through which the fluid to be discharged through the discharge hole 15a of the lesion indicating part 11a flows. The fluid channel 25a is partitioned into a lower fluid channel 27 and an upper fluid channel 29 by a partition plate 35.

The module body 21a has an inlet 31a and an outlet 33a formed therein. The fluid is introduced into the lower fluid channel 27 through the inlet 31a and discharged from the lower fluid channel 27 through the outlet 33a. The inlet 31a and the outlet 33a are connected by a non-illustrated tube.

The partition plate 35 has an upper inlet 37 formed therein, through which part of the fluid flowing through the lower fluid channel 27 is introduced into the upper fluid channel 29. Part of the fluid flowing through the upper fluid channel 29 is discharged through the discharge hole 15a of the lesion indicating part 11a. Accordingly, the lesion indicating part 11a presents a bleeding state.

The spacing space 41 is concavely formed to a predetermined depth on a surface of the central region of the partition plate 35 that faces the lesion indicating part 11a. The spacing space 41 is preferably formed in a position corresponding to the syringe needle passage portion 17.

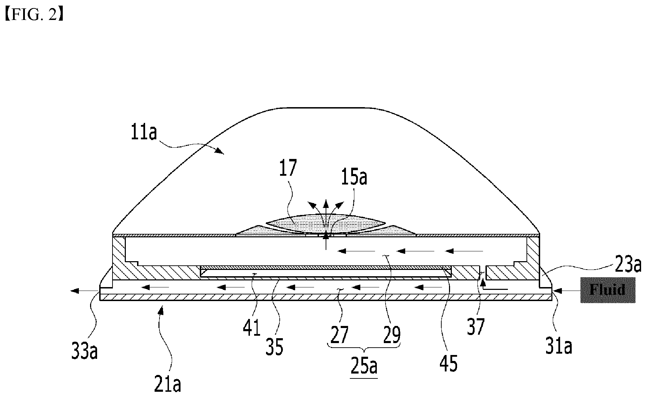

The diaphragm 45 is provided in the spacing space 41 of the module body 21a so as to be expandable such that the diaphragm 45 is separated from the upper fluid channel 29. Accordingly, the spacing space 41 between the partition plate 35 of the module body 21a and the diaphragm 45 forms an empty space as illustrated in FIG. 2.

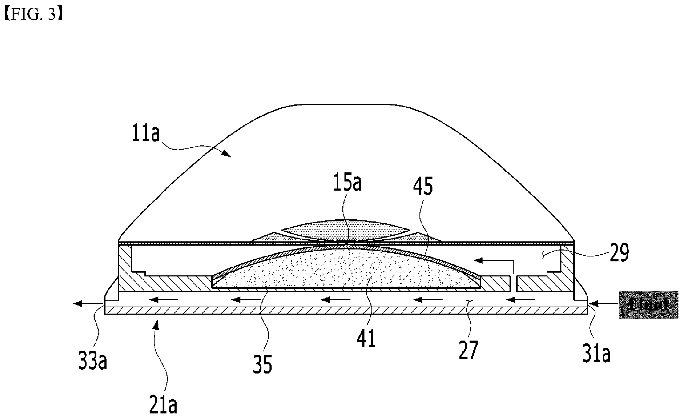

When the separate fluid distinct from the fluid flowing through the upper fluid channel 29 is injected into the spacing space 41 through the syringe needle via the syringe needle passage portion 17 of the lesion indicating part 11a and the diaphragm 45, if the amount of the separate fluid injected exceeds the limited volume of the spacing space 41, the diaphragm 45 expands toward the lesion indicating part 11a and closes the discharge hole 15a of the lesion indicating part 11a as illustrated in FIG. 3, and an effect of stanching a bleeding part is presented.

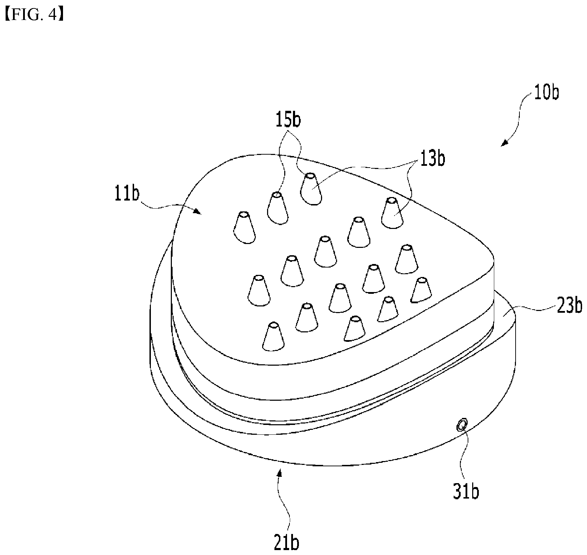

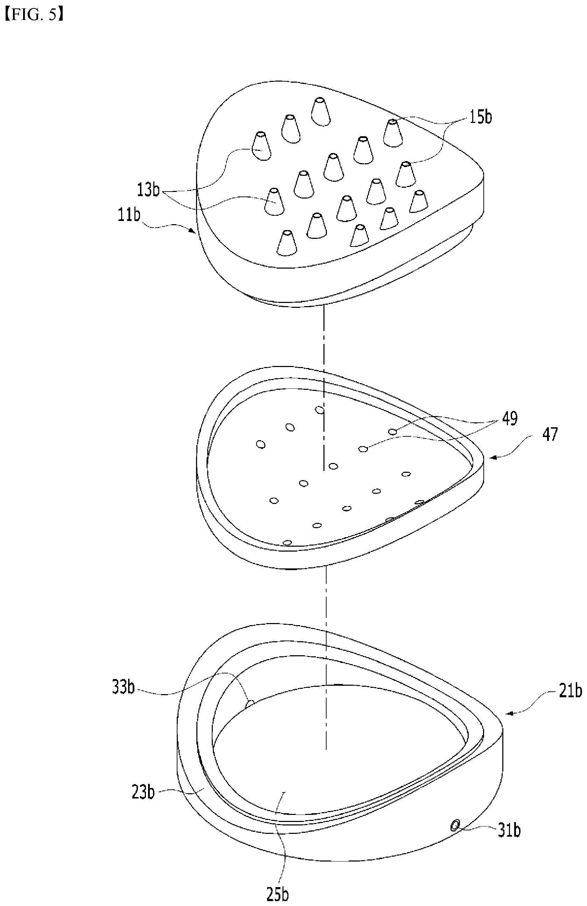

FIGS. 4 to 6 illustrate an endoscopic procedure simulator module according to a second embodiment of the inventive concept.

As illustrated in these drawings, the endoscopic procedure simulator module 10b according to the second embodiment of the inventive concept includes a lesion indicating part 11b and a module body 21b.

The lesion indicating part 11b has a disc shape that is concavely formed to be curved to one side. The lesion indicating part 11b has a plurality of protrusions 13b protruding from a plate surface thereof. Furthermore, each of the protrusions 13b has a discharge hole 15b formed therein, through which a fluid is discharged. Accordingly, as the fluid is discharged through the discharge holes 15b, the lesion indicating part 11b may not only present formation of a plurality of polyps, but may also present a bleeding state through the polyps. Although the lesion indicating part 11b in this embodiment is illustrated as being concavely formed to be curved to the one side, the lesion indicating part 11b, without being limited thereto, may protrude so as to be curved to the one side or may be formed to be flat.

The module body 21b has a disc shape. The module body 21b has, on the periphery thereof, a coupling part 23b with a reduced diameter. The coupling part 23b is fit into the coupling hole 117 of the endoscopic procedure simulator 100, which will be described below. Accordingly, the module body 21b is coupled to the endoscopic procedure simulator 100.

The module body 21b has a fluid channel 25b formed therein, through which the fluid to be discharged through the discharge holes 15b of the lesion indicating part 11b flows.

The module body 21b has an inlet 31b and an outlet 33b formed therein. The fluid is introduced into the fluid channel 25b through the inlet 31b and discharged from the fluid channel 25b through the outlet 33b. The inlet 31b and the outlet 33b are connected by a non-illustrated tube.

The endoscopic procedure simulator module 10b according to the second embodiment of the inventive concept further includes a distribution member 47.

The distribution member 47 is provided between the lesion indicating part 11b and the module body 21b. The distribution member 47 has a plurality of distribution holes 49 formed through the distribution member 47. The distribution holes 49 distribute and supply the fluid that is discharged from the fluid channel 25b of the module body 21b to the discharge holes 15b of the plurality of protrusions 13b. The distribution holes 49 communicate with the fluid channel 25b of the module body 21b and the discharge holes 15b.

The endoscopic procedure simulator module 10b according to the second embodiment of the inventive concept has a structure in which the module body 21b, the distribution member 47, and the lesion indicating part 11b are fit into each other and sequentially stacked on each other.

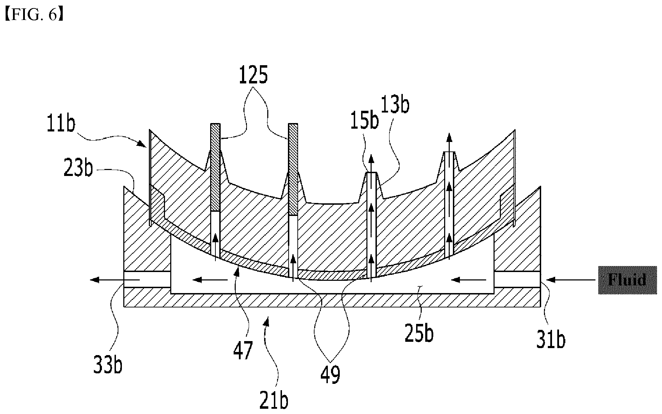

The endoscopic procedure simulator module 10b according to the second embodiment of the inventive concept, as partly illustrated in FIG. 6, presents an effect of stanching a bleeding part of the polyp when a pin 125 is inserted into the discharge hole 15b of the protrusion 13b through which the fluid is discharged.

The endoscopic procedure simulator module 10b according to the second embodiment of the inventive concept is illustrated as having the configuration in which the module body 21b, the distribution member 47, and the lesion indicating part 11b are separable from each other and are fit into each other. However, without being limited thereto, the module body 21b, the distribution member 47, and the lesion indicating part 11b may be implemented in one integrated form by using a 3D printer, without being separated from each other.

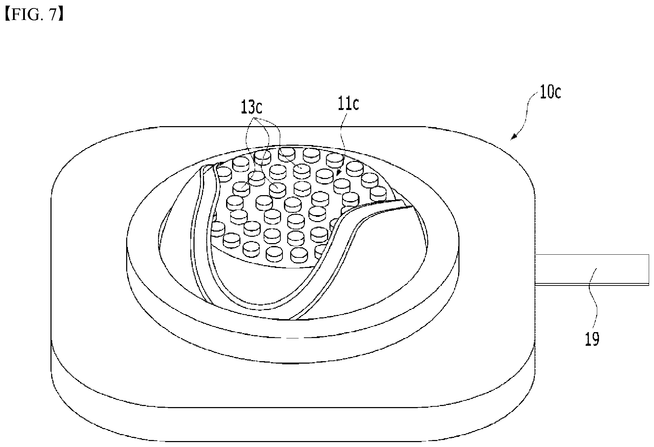

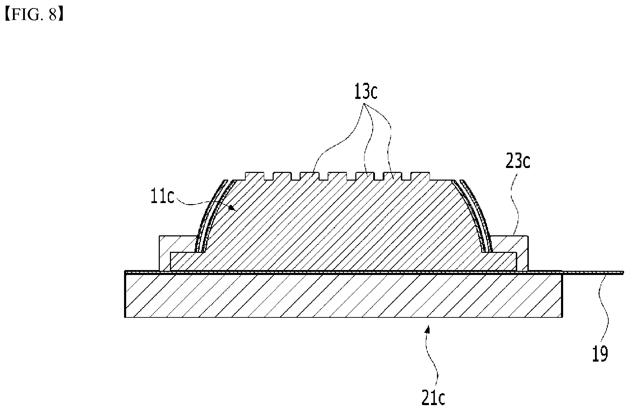

FIGS. 7 and 8 illustrate an endoscopic procedure simulator module according to a third embodiment of the inventive concept.

Unlike the endoscopic procedure simulator modules 10a and 10b in the above-described embodiments, the endoscopic procedure simulator module 10c according to the third embodiment of the inventive concept includes a lesion indicating part 11c having a hemispherical shape and a plurality of protrusions 13c that protrude from the surface of the lesion indicating part 11c to present polyps. A lower end portion of the lesion indicating part 11c protrudes to form a step along the circumferential direction so as not to be separated from a module body 21c.

The lesion indicating part 11c is formed of a conductive material. The lesion indicating part 11c is preferably formed in a gel form containing polyvinyl alcohol.

A terminal 19 that can conduct electricity is provided on the entire plate surface of a lower module body that faces an upper module body including a bottom surface of the lesion indicating part 11c. One side of the terminal 19 protrudes from the module body 21c and acts as an electrode. An electric wire is electrically coupled to the protruding portion of the terminal 19.

The module body 21c includes the lower module body and the upper module body. The lower module body has a rectangular block shape, and the lesion indicating part 11c and the terminal 19 are seated on the lower module body. The upper module body has an arc shape to receive the lesion indicating part 11c therein and protrudes from the lower module body. The upper module body receives the lesion indicating part 11c therein such that the plurality of protrusions 13c are exposed. The protrusions 13c of the lesion indicating part 11c are located in a higher position than the edge of the upper module body.

A coupling part 23c protruding while forming a step with the lower module body is formed around a lower portion of the upper module body. The coupling part 23c is fit into the coupling hole 117 of the endoscopic procedure simulator 100, which will be described below. Accordingly, the module body 21c is coupled to the endoscopic procedure simulator 100. The module body 21c is formed of an insulating material that does not conduct electricity.

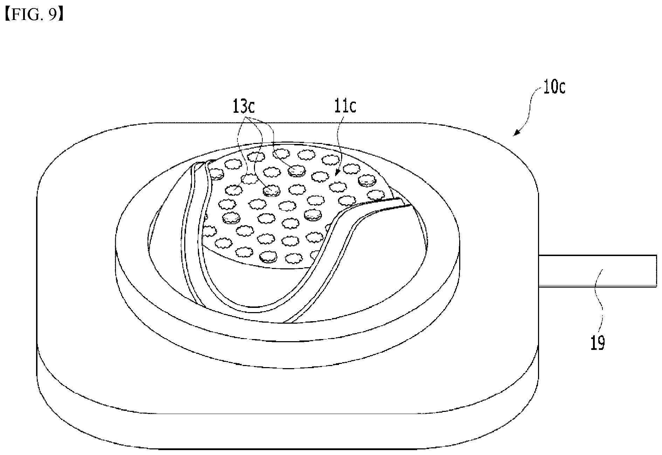

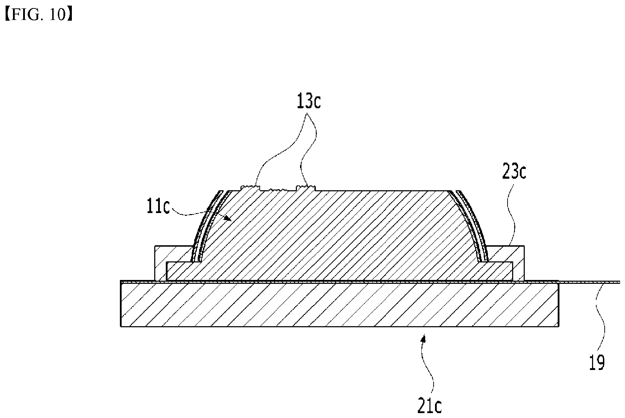

When electricity is supplied through the terminal 19, which is connected to the electric wire, to energize the lesion indicating part 11c and an electric knife (not illustrated) is brought into contact with the protrusions 13c, sparks are generated between the electric knife and the protrusions 13c of the lesion indicating part 11c, and the protrusions 13c are melted as illustrated in FIGS. 9 and 10. Accordingly, an effect of removing the polyps is presented as the protrusions 13c are melted while generating heat.

The endoscopic procedure simulator module 10c according to the third embodiment of the inventive concept is preferably frozen in a freezer or immersed in a saline solution to prevent the lesion indicating part 11c from being dried.

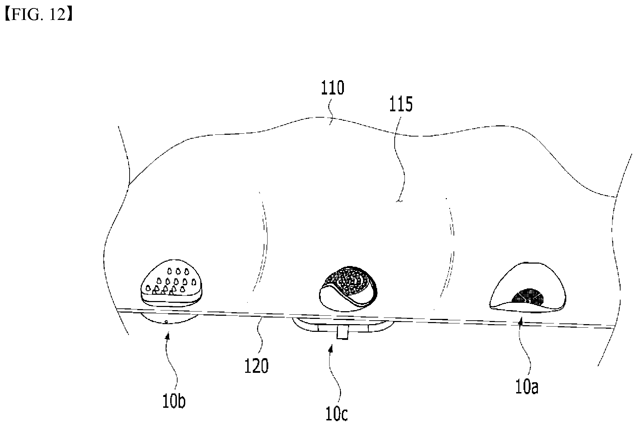

FIGS. 11 and 12 illustrate an endoscopic procedure simulator according to an embodiment of the inventive concept.

The endoscopic procedure simulator 100 according to the embodiment of the inventive concept includes a model organ 110 and the modules 10a, 10b, and 10c.

The model organ 110 has a simulated organ shape. The model organ 110 in a stomach shape connected to a throat connected to an oral cavity (not illustrated) in a human body is illustrated in this embodiment. However, without being limited thereto, the model organ 110 may have the shape of an organ such as a small intestine, a large intestine, an anus, or the like. For example, the model organ 110 according to the inventive concept may be formed to be the same as an actual body structure, thereby enabling realistic endoscopic procedure training.

The model organ 110 has an insertion space 115 formed therein, and an endoscope (not illustrated) is inserted and moved into the insertion space 115. Furthermore, the model organ 110 has a plurality of coupling holes 117 that are formed through the surface of the model organ 110 and that communicate with the insertion space 115. In this embodiment, the plurality of coupling holes 117 are illustrated as being formed through the model organ 110. However, only one coupling hole 117 may be formed through the model organ 110.

The plurality of coupling holes 117 to which the modules 10a, 10b, and 10c according to the first to third embodiments described above are selectively detachably coupled are formed through the model organ 110. The coupling holes 117 are formed through the surface of the model organ 110 so as to communicate with the insertion space 115. The coupling holes 117 have diameters by which the coupling parts 23a, 23b, and 23c of the modules 10a, 10b, and 10c are fit into the coupling holes 117.

The model organ 110 is formed of a soft material to allow an operator to feel a sense of an actual organ when manipulating the endoscope and performing an endoscopic procedure. The model organ 110 is preferably formed of one of silicone, vinyl chloride, and urethane. The model organ 110 may be integrally injection molded. Alternatively, the model organ 110 may be formed by connecting upper and lower injection-molded parts. In another case, the model organ 110 may be formed by connecting injection-molded parts with a predetermined length. Furthermore, the model organ 110 may be manufactured by using a mold capable of molding a soft material to correspond to the interior of the mold formed of a hard material.

The modules 10a, 10b, and 10c according to the first to third embodiments described above are provided as modules. The modules 10a, 10b, and 10c are fit into the coupling holes 117 of the model organ 110 such that the lesion indicating parts 11a, 11b, and 11c are exposed to the insertion space 115 as illustrated in FIG. 12. Furthermore, the modules 10a, 10b, and 10c according to the first to third embodiments described above may be selectively coupled to the plurality of coupling holes 117 of the model organ 110 while changing the positions of the coupling holes 117, thereby presenting various lesion phenomena in various positions.

The endoscopic procedure simulator 100 according to the embodiment of the inventive concept may further include a fixing frame 120 for surrounding and fixing the model organ 110.

The fixing frame 120 may be formed of a material with a higher hardness than the model organ 110 to maintain the shape of the model organ 110 and stably mount the model organ 110 on the floor. The fixing frame 120 may be integrally injection molded. Alternatively, the fixing frame 120 may be formed by connecting upper and lower injection-molded parts. In another case, the fixing frame 120 may be formed by connecting injection-molded parts with a predetermined length.

Through-holes (not illustrated) that communicate with the coupling holes 117 are formed through the fixing frame 120 to correspond to the coupling holes 117 of the model organ 110. The modules 10a, 10b, and 10c fit into the coupling holes 117 of the model organ 110 are fit into the through-holes.

The endoscopic procedure simulator 100 according to the embodiment of the inventive concept is illustrated in FIG. 11 in the state in which part of the fixing frame 120 is removed to expose the model organ 110. However, this is only to help with comprehension of the inventive concept, and the fixing frame 120 may surround and fix the outside of the model organ 110 so as not to expose the model organ 110.

A description of an endoscopic procedure training process in the state in which the modules 10a, 10b, and 10c according to the first to third embodiments described above are mounted in the respective coupling holes 117 of the model organ 110 will be given below.

Hereinafter, for convenience of description, the module according to the first embodiment described above is referred to as a first module 10a, the module according to the second embodiment described above is referred to as a second module 10b, and the module according to the third embodiment described above is referred to as a third module 10c.

First, tubes (not illustrated) are connected to the inlets 31a and 31b and the outlets 33a and 33b of the first and second modules 10a and 10b, and a fluid in a liquid phase is injected through the inlets 31a and 31b of the first and second modules 10a and 10b. Simultaneously, a power source is connected to the terminal 19 of the third module 10c to energize the lesion indicating part 11c of the third module 10c.

Accordingly, as the fluid is discharged through the discharge hole 15a of the lesion indicating part 11a, the first module 10a presents a state in which blood is lost from an internal mucous membrane of a organ. As the fluid is discharged through the discharge hole 15a of the lesion indicating part 11a, the second module 10b presents a state in which blood is lost from a polyp formed on the internal mucous membrane of the organ. The third module 10c presents a state in which the polyp is formed on the internal mucous membrane of the organ.

Next, an endoscope is inserted through an oral cavity (not illustrated) of the model organ 110 located on a right side of FIG. 11 and is moved along the insertion space 115 of the model organ 100 via a throat part. The endoscope includes a camera channel into which a camera is inserted, a working channel into which a syringe needle and an electric knife for cutting a lesion are inserted and moved, and a suction channel for removing foreign matter generated from a diseased part.

At this time, the endoscope is moved along the insertion space 115 while the interior of the model organ 110 is examined through the camera of the endoscope.

When the endoscope reaches each of the modules 10a, 10b, and 10c that present lesion states, a procedure trainee performs endoscopic procedure training while manipulating the endoscope.

Hereinafter, endoscopic procedure training processes for lesion phenomena in the respective modules 10a, 10b, and 10c will be described.

In the case of the first module 10a that presents the state in which blood is lost from the internal mucous membrane of the organ, a fluid, for example, a saline solution different from the fluid flowing through the upper fluid channel 29 of the first module 10a is injected into the spacing space 41 through the syringe needle inserted into the working channel of the endoscope, via the syringe needle passage portion 17 of the lesion indicating part 11a and the diaphragm 45.

When the saline solution is injected into the spacing space 41 through the syringe needle, the diaphragm 45 expands toward the lesion indicating part 11a if the amount of the saline solution injected exceeds the limited volume of the spacing space 41.

A procedure for stanching a bleeding part on the internal mucous membrane of the organ may be implemented by injecting the saline solution into the spacing space 41 such that the discharge hole 15a of the lesion indicating part 11a is closed as illustrated in FIG. 3.

In the case of the second module 10b that presents the state in which blood is lost from the polyp formed on the internal mucous membrane of the organ, a procedure for stanching a bleeding part of the polyp may be implemented as partly illustrated in FIG. 6, by closing the discharge hole 15b, through which the fluid is discharged, by inserting the pin 125 (refer to FIG. 6) into the discharge hole 15b of the protrusion 13b using a procedure instrument, such as a catheter, which is inserted into the working channel of the endoscope.

In the case of the third module 10c that presents the state in which the polyp is formed on the internal mucous membrane of the organ, when the electric knife inserted into the working channel of the endoscope is brought into contact with the protrusions 13c to be treated, sparks are generated between the electric knife and the protrusions 13c of the lesion indicating part 11c, and the protrusions 13c are melted. Accordingly, a procedure for removing the polyp may be implemented as illustrated in FIGS. 9 and 10.

As described above, various lesion phenomena may be presented through the modules detachably coupled to the model organ 110, the endoscope may be inserted and moved along the insertion space 115 of the model organ 110, and training in endoscope manipulation and endoscopic procedure may be repeatedly performed in response to the various lesion phenomena presented by the modules.

The endoscopic procedure simulator modules according to the embodiments described above and the endoscopic procedure simulator may be injection molded or may be manufactured by using a 3D printer.

According to the inventive concept, the endoscopic procedure simulator modules enable repeated training in endoscope manipulation and endoscopic procedure in response to various lesion phenomena.

While the inventive concept has been described with reference to exemplary embodiments, it will be apparent to those skilled in the art that various changes and modifications may be made.

## Claims

1. An endoscopic procedure simulator module comprising:
a lesion indicating part (11a) having a discharge hole (15a) formed therein, through which a fluid is discharged to present a bleeding state;
a module body (21a) to which the lesion indicating part (11a) is coupled, the module body (21a) having a fluid channel (25a) and a spacing space (41) formed therein, wherein the fluid to be discharged through the discharge hole (15a) flows through the fluid channel (25a) and the spacing space (41) is separated from the fluid channel (25a); and
a diaphragm (45) provided in the spacing space (41) of the module body (21a) so as to be expandable, wherein the diaphragm (45) is expanded by a separate fluid injected into the spacing space (41) and closes the discharge hole (15a) of the lesion indicating part (11a),
wherein the lesion indicating part (11a) has a syringe needle passage portion (17) that is formed around the discharge hole (15a) thereof and through which a syringe needle configured to inject the separate fluid into the spacing space (41) passes.

2. The endoscopic procedure simulator module of claim 1, wherein the fluid channel (25a) is partitioned into a lower fluid channel (27) and an upper fluid channel (29) by a partition plate (35), the partition plate (35) having an upper inlet (31a) formed therein, through which part of the fluid flowing through the lower fluid channel (27) is introduced into the upper fluid channel (29).

3. The endoscopic procedure simulator module of claim 2, wherein the module body (21a) has an inlet (31a) and an outlet (33a) that are formed therein and connected by a tube, the fluid being introduced into the lower fluid channel (27) through the inlet (31a) and discharged from the lower fluid channel (27) through the outlet (33a).

4. An endoscopic procedure simulator comprising:
a model organ having the shape of an organ and including an insertion space (115) formed therein and one or more coupling holes (117) formed through a surface thereof, wherein an endoscope is inserted and moved into the insertion space (115) and the one or more coupling holes (117) communicate with the insertion space (115); and
the module set forth in any one of claims 1 to 4, the module being detachably coupled to the coupling hole (117) of the model organ such that the lesion indicating part (11a, 11b, 11c) is exposed to the insertion space (115).

5. The endoscopic procedure simulator of claim 4, further comprising:
a fixing frame (120) configured to surround and fix the model organ.

## Patentansprüche

1. Simulatormodul für endoskopisches Verfahren, umfassend:
einen Läsionsanzeigeteil (11a) mit einem darin ausgebildeten Ablaufloch (15a), durch das hindurch eine Flüssigkeit abläuft und so einen Blutungszustand darstellt;
einen Modulkörper (21a), mit dem der Läsionsanzeigeteil (11a) verbunden ist, wobei der Modulkörper (21a) einen Flüssigkeitskanal (25a) und einen darin ausgebildeten Abstandszwischenraum (41) aufweist, wobei die durch das Ablaufloch (15a) abzulassende Flüssigkeit durch den Flüssigkeitskanal (25a) fließt und der Abstandszwischenraum (41) vom Flüssigkeitskanal (25a) getrennt ist; und
eine Membran (45), die im Abstandszwischenraum (41) des Modulkörpers (21a) so angeordnet ist, dass sie ausdehnbar ist, wobei die Membran (45) mit einer separaten Flüssigkeit ausgedehnt wird, die in den Abstandszwischenraum (41) eingespritzt wird, und das Ablaufloch (15a) des Läsionsanzeigeteils (11a) verschließt,
wobei der Läsionsanzeigeteil (11a) einen Spritzennadel-Durchführungsabschnitt (17) aufweist, der um das Ablaufloch (15a) desselben ausgebildet ist und durch den hindurch eine Spritzennadel geführt wird, die so eingerichtet ist, dass sie die separate Flüssigkeit in den Abstandszwischenraum (41) einspritzt.

2. Simulatormodul für endoskopisches Verfahren nach Anspruch 1, wobei der Flüssigkeitskanal (25a) mit einer Trennplatte (35) in einen unteren Flüssigkeitskanal (27) und einen oberen Flüssigkeitskanal (29) getrennt ist, wobei die Trennplatte (35) einen darin ausgebildeten oberen Einlass (31a) aufweist, durch den hindurch ein Teil der Flüssigkeit, die durch den unteren Flüssigkeitskanal (27) fließt, in den oberen Flüssigkeitskanal (29) geleitet wird.

3. Simulatormodul für endoskopisches Verfahren nach Anspruch 2, wobei der Modulkörper (21a) einen Einlass (31a) und einen Auslass (33a) aufweist, die darin ausgebildet und über eine Röhre verbunden sind, wobei die Flüssigkeit durch den Einlass (31a) in den unteren Flüssigkeitskanal (27) geleitet wird und durch den Auslass (33a) aus dem unteren Flüssigkeitskanal (27) abläuft.

4. Simulator für endoskopisches Verfahren, umfassend:
ein Modellorgan mit der Form eines Organs, das einen darin ausgebildeten Einführraum (115) und ein oder mehrere Verbindungslöcher (117) aufweist, die durch eine Oberfläche davon ausgebildet sind, wobei ein Endoskop in den Einführraum (115) eingeführt und in ihn hinein bewegt wird und das eine oder die mehreren Verbindungslöcher (117) mit dem Einführraum (115) in Verbindung stehen; und
das in einem der Ansprüche 1 bis 4 dargelegte Modul, wobei das Modul mit dem Verbindungsloch (117) des Modellorgans derart lösbar verbunden ist, dass der Läsionsanzeigeteil (11a, 11b, 11c) zum Einführraum (115) hin freiliegt.

5. Simulator für endoskopisches Verfahren nach Anspruch 4, ferner umfassend:
einen Fixierrahmen (120), der so eingerichtet ist, dass er das Modellorgan umgibt und fixiert.

## Revendications

1. Un module formant simulateur pour procédure endoscopique comprenant :
une partie (11a) d'indication de lésion ayant un trou de décharge (15a) formé à l'intérieur d'elle, à travers lequel un fluide est déchargé de façon à présenter un état de saignement ;
un corps de module (21a) auquel la partie (11a) d'indication de lésion est reliée, le corps de module (21a) ayant un canal de fluide (25a) et un espace d'espacement (41) formé à l'intérieur de lui, le fluide qui doit être évacué par le trou de décharge (15a) s'écoulant à travers le canal de fluide (25a) et l'espace d'espacement (41) étant séparé du canal de fluide (25a) ; et
un diaphragme (45) prévu dans l'espace d'espacement (41) du corps de module (21a) de manière à être extensible, le diaphragme (45) étant dilaté par un fluide séparé injecté dans l'espace d'espacement (41) et fermant le trou de décharge (15a) de la partie (11a) d'indication de lésion,
la partie (11a) d'indication de lésion ayant une partie (17) de passage d'aiguille de seringue qui est formée autour du trou de décharge (15a) qu'elle comprend et à travers laquelle une aiguille de seringue configurée pour injecter le fluide séparé passe dans l'espace d'espacement (41).

2. Le module formant simulateur pour procédure endoscopique selon la revendication 1, dans lequel le canal de fluide (25a) est divisé en un canal de fluide inférieur (27) et un canal de fluide supérieur (29) par une plaque de séparation (35), la plaque de séparation (35) ayant une entrée supérieure (31a) formée à l'intérieur d'elle, à travers laquelle une partie du fluide s'écoulant à travers le canal de fluide inférieur (27) est introduite dans le canal de fluide supérieur (29).

3. Le module formant simulateur pour procédure endoscopique selon la revendication 2, dans lequel le corps de module (21a) a une entrée (31a) et une sortie (33a) qui sont formées en lui et qui sont reliées par un tube, le fluide étant introduit dans le canal de fluide inférieur (27) par l'entrée (31a) et étant évacué du canal de fluide inférieur (27) par la sortie (33a).

4. Un simulateur pour procédure endoscopique comprenant :
un organe modèle ayant la forme d'un organe et comprenant un espace d'insertion (115) formé à l'intérieur de lui et un ou plusieurs trous de liaison (117) formés à travers une surface de celui-ci, un endoscope étant inséré et déplacé dans l'espace d'insertion (115) et lesdits un ou plusieurs trous de liaison (117) communiquant avec l'espace d'insertion (115) ; et
le module selon l'une quelconque des revendications 1 à 4, le module étant relié de manière amovible au trou de liaison (117) de l'organe modèle de telle sorte que la partie (11a, 11b, 11c) d'indication de lésion soit exposée à l'espace d'insertion (115).

5. Le simulateur pour procédure endoscopique selon la revendication 4, comprenant en outre :
un châssis de fixation (120) configuré pour entourer et fixer l'organe modèle.
